Europäisches Patentamt

European Patent Office

Office européen des brevets

(11) Numéro de publication: **0 206 852**

**A1**

(12) # DEMANDE DE BREVET EUROPEEN

(21) Numéro de dépôt: **86401020.2**

(51) Int. Cl.⁴: **A61K 39/09**

(22) Date de dépôt: **13.05.86**

Le titre de l'invention a t modifi (Directives l'examen pratiqu l'OEB, A-III, 7.3)

(30) Priorité: **14.05.85 FR 8507315**

(43) Date de publication de la demande:
**30.12.86 Bulletin 86/52**

(84) Etats contractants désignés:
**AT BE CH DE FR GB IT LI LU NL SE**

(71) Demandeur: **UNIVERSITE CATHOLIQUE DE LOUVAIN**
**Place de l'Université, 1**
**B-1348 Louvain la Neuve(BE)**
Demandeur: **INSTITUT NATIONAL DE LA SANTE ET DE LA RECHERCHE MEDICALE (INSERM)**
**101, rue de Tolbiac**
**F-75654 Paris Cedex 13(FR)**

(72) Inventeur: **Frank, Robert**
**12, rue Silbermann**
**F-67000 Strasbourg(FR)**
Inventeur: **Klein, Jean-Paul**
**19, rue Albert Schweitzer**
**F-67112 Breuschwickersheim(FR)**
Inventeur: **Ackermans, Fabienne**
**12, avenue de l'Oiseau bleu**
**B-1150 Bruxelles(BE)**
Inventeur: **Bazin, Hervé**
**Avenue des Eglantines**
**B-741150 Bruxelles(BE)**

(74) Mandataire: **Ayache, Monique et al**
**CABINET PLASSERAUD 84, rue d'Amsterdam**
**F-75009 Paris(FR)**

(54) **Conjugué constitué d'une adhésine de paroi de Streptococcus mutans, de nature protéique et d'un polysaccharide de Streptococcus mutans, sa préparation et son utilisation notamment dans des vaccins anti-caries.**

(57) Le conjugué selon l'invention est constitué d'une adhésine de paroi de Streptococcus mutans, de nature protéique, capable de lier les composants de la salive, ou d'une fraction active d'une telle protéine, couplée de façon covalente à un polysaccharide de Streptococcus mutans, ou à une fraction d'un tel polysaccharide.

De préférence, l'adhésine est constituée d'une chaîne simple polypeptidique ayant un poids moléculaire de 74 000 environ; elles est commune aux différents sérotypes de Streptococcus mutans où elle présente une antigénicité croisée ; et elle est capable d'interagir avec les glycoprotéines salivaires et le polysaccharide provient du même sérotype de S. mutans, notamment le sérotype f.

Application à la préparation de compositions immunogènes, notamment à la préparation de vaccins
contre la carie dentaire.

## Conjugué constitué d'une adhésine de paroi de S. mutans, de nature protéique et d'un polysaccharide de S. mutans, sa préparation et son utilisation notamment dans des vaccins anti-caries.

L'invention concerne un produit obtenu par liaison convalente d'une protéine avec un polysaccharide, sa préparation et son utilisation.

Plus précisément, l'invention a pour objet un produit dans lequel une adhésine de paroi de Streptococcus mutans, de nature protéique, ou une fraction d'une telle protéine, est liée de façon covalente à un polysaccharide de Streptococcus mutans, ou à une fraction d'un tel polysaccharide, un procédé pour sa préparation et son utilisation comme antigène, notamment dans des vaccins contre les caries dentaires.

Streptoccus mutans est considéré à l'heure actuelle comme étant la principale bactérie responsable de la maladie carieuse chez l'Homme et chez l'Animal. Cette espèce bactérienne est formée de 8 sérotypes différents portant les références a à h.

Au cours des dernières années, de nombreuses expériences d'immunisation, utilisant comme antigène cette bactérie, ont montré la possibilité d'obtenir une protection contre les lésions carieuses, au moyen d'un tel antigène. Toutefois l'utilisation de cette bactérie complète semble conduire à des réactions croisées indésirables avec d'autres tissus tels que le tissu cardiaque, c'est pourquoi les travaux actuels sont principalement orientés vers la recherche d'antigènes simples, dérivés de cette bactérie, et possédant le même protecteur.

Les caractéristiques principales du Streptococcus mutans sont de pouvoir adhérer à la pellicule exogène acquise formée à la surface de la dent principalement par des glycoprotéines salivaires adsorbées, et ceci par l'intermédiaire d'adhésines de paroi de nature protéique ou polysaccharidique, et de synthétiser, grâce à des enzymes, les glucosyltransférases (GTF), des dextranes qui assurent la cohésion des bactéries au niveau de la plaque dentaire.

La possibilité d'inhiber la fixation et l'agrégation du Streptoccus mutans sur la dent a été étudiée par de nombreux auteurs au cours d'expériences d'immunisation utilisant les GTF comme antigènes. Toutefois les résultats obtenus ne permettent pas de conclure à l'efficacité d'un tel antigène, peut-être en raison du très grand polymorphisme des GTF de Streptoccus mutans.

En revanche, l'utilisation de certaines protéines purifiées à partir des surnageants de Streptococcus mutans, c'est-à-dire excrétées par cette bactérie, dont le rôle biologique n'est pas connu, ont donné lieu à des résultats encourageants au cours d'expériences d'immunisations animales.

Une autre possibilité d'agir sur la colonisation des surfaces dentaires serait de bloquer l'adhérence de Streptoccus mutans en induisant la synthèse d'anticorps contre les adhésines de paroi capables d'interagir avec les glycoprotéines salivaires.

Cette solution a déjà été envisagée mais n'a pas, jusqu'à présent, conduit à des résultats satisfaisants. Ainsi il a été observé que les polysaccharides des différents sérotypes, bien qu'intervenant dans le processus d'adhérence, sont pratiquement incapables d'induire des réponses immunitaires durables.

Certains auteurs ont en outre proposé d'utiliser comme antigènes des adhésines de paroi de nature protéique; il en est ainsi par exemple dans la demande de brevet EP 116 472. Des résultats plus encourageants ont été ainsi obtenus.

L'invention a pour but de mettre au point un produit comprenant une adhésine de paroi de Streptococcus mutans, de nature protéique, capable d'induire des réponses immunitaires plus fortes et/ou plus durables que celles qui sont induites par l'adhésine elle-même.

Ce but a été atteint selon l'invention qui a notamment pour objet un conjugué constitué d'une adhésine de paroi de Streptococcus mutans de nature protéique, capable de lier les composants de la salive, ou d'une fraction active d'une telle protéine, couplée de façon covalente à un polysaccharide de Streptococcus mutans, ou à une fraction d'un tel polysaccharide.

La liaison covalente est effectuée selon des méthodes connues en soi, utilisables en biologie et compatibles avec la structure de la protéine d'une part et du polysaccharide d'autre part. Elle peut notamment être effectuée par l'intermédiaire d'un "bras" introduit de manière connue en soi et faisant intervenir des réactifs bifonctionnels tels que les carbodiimides par exemple.

Toutefois, le "bras" inséré dans la structure du produit pouvant éventuellement conduire à la formation d'anticorps spécifiques indésirables, il peut être préférable de ne pas y avoir recours pour établir la liaison covalente.

Selon un mode préféré de réalisation, la liaison covalente est effectuée par oxydation ménagée du polysaccharide, notamment par le periodate de sodium, suivie d'un couplage avec la protéine par animation réductrice en présence de borohydrure de sodium.

L'adhésine de paroi de nature protéique qui est contenue dans l'extrait de paroi cellulaire des bactéries (cell Wall Extracted Antigen: WEA) peut être obtenue selon toute méthode décrite dans la littérature, en particulier selon la méthode de M. Schöller et coll. décrite dans Infect.Immun.31, 52-60 (1981) ou par analogie à cette methode, par exemple comme décrit par J. A. Ogier et coll. dans Infect. Immun. 45(1), 107-112 (1984).

Il peut être également avantageux de n'utiliser qu'une fraction d'une telle adhésine, c'est-à-dire une fraction capable de lier les composants de la salive et d'induire des anticorps, obtenue par fractionnement d'une adhésine préparée comme indiqué plus haut, par synthèse chimique ou encore par génie génétique.

Le polysaccharide de Streptococcus mutans peut être obtenu notamment selon la méthode de S. Hamada et coll. décrite dans Molec. Immunol. 20, 453 (1983).

Il peut être également avantageux de n'utiliser qu'une fraction active d'un tel polysaccharide, c'est-à-dire une fraction capable notamment de lier les composants de la salive.

L'adhésine de paroi de nature protéique, ou sa fraction, utilisée provient de préférence du même sérotype que le polysaccharide ou la fraction de polysaccharide utilisée. Toutefois, compte tenu de l'antigénicité croisée observée avec la plupart des adhésines de nature protéique, il est également possible de combiner une adhésine et un polysaccharide provenant de sérotypes différents, ou des fractions de ceux-ci.

Le conjugué ainsi obtenu, notamment lorsqu'il est administré par voie orale à des animaux, en particulier en association avec des liposomes, conduit à des réponses en IgA dans la salive et le sérum supérieures à celles obtenues avec la protéine non couplée. Il est rappelé à ce propos que les IgA sont des immunoglobulines capables de protéger contre les caries dentaires [voir M. A. Taubman et coll. dans J. Dent. Res. 60, 634 (1981)].

Le conjugué selon l'invention est en outre capable d'induire une réponse locale et systémique en IgA dirigée contre le polysaccharide utilisé alors que le même polysaccharide, administré dans les mêmes conditions, notamment en association avec des liposomes, est incapable d'induire une quelconque réponse en IgA.

Ceci est d'autant plus surprenant qu'il avait été montré, de façon générale, que la conjugaison de différemts polysaccharides bactériens avec des protéines, selon différentes méthodes, augmentait leur immunogénicité en induisant une réponse en IgG anti-polysaccharides [voir par exemple E. C.

Beuvery et coll., Infect. Immun.40, 39 (1983) et 0. Mäkelä et coll., Scand. J. Immunol, 19, 541 (1984)-], alors qu'avec le conjugué selon l'invention la réponse en IgG est faible.

Enfin, le conjugué selon l'invention confère, tout comme son constituant protéique, une "mémoire immunologique", alors que le polysaccharide seul est dénué de cette propriété.

Compte tenu de l'ensemble de ses propriétés, le conjugué selon l'invention peut être utilisé dans diverses applications en tant qu'antigène, et en particulier en tant que composé à activité immuniogène. De plus, et surtout, il peut être utilisé, seul ou en mélange avec d'autres antigènes du même type ou avec des adhésines de paroi de Streptococcus mutans, notamment de nature protéique, dans des vaccins contre la carie dentaire.

Compte tenu des différents sérotypes de Streptococcus mutans existants, il peut être avantageux d'utiliser ensemble différents conjugués dans lesquels au moins les adhésines de paroi de nature protéique proviennent de sérotypes différents de Streptococcus mutans.

Selon un mode avantageux de réalisation, le conjugué selon l'invention comprend une adhésine de paroi de nature protéique, nouvellement isolée, appelée dans ce qui suit 74K SR(SR = Saliva Receptor: récepteur de la salive), qui est caractérisée en ce que :

-elle est constituée d'une chaîne simple polypeptidique ayant un poids moléculaire de 74 000 environ;

-elle est commune aux différents sérotypes de Streptococcus mutansoù elle présente une antigénicité croisée ; et

-elle est capable d'interagir avec les glycoprotéines salivaires.

Cette protéine peut être préparée à partir d'une souche de S. mutans de sérotype f, d'origine humaine, par extraction des antigènes bruts associés à la paroi cellulaire des bactéries, au moyen de tampon phosphate 0,5 M, pH 6,0 et purification par chromatographie d'immunoaffinité au moyen d'un anticorps monoclonal dirigé contre les protéines interagissant avec les glycoprotéines salivaires, en présence d'un détergent non ionique et de dodécylsulphate de sodium, avec élution par du tampon NcCl ou glycine/HCl.

Les détails de cette préparation seront donnés dans les exemples qui suivent.

L'antigène 74K SR, de nature protéique, est un produit nouveau capable à lui seul d'induire une réponse locale et systémique en IgA et de conférer une "mémoire immunologique". Il peut donc être utilisé comme principe actif dans des compositions à activité immunogène, en particulier dans des vaccins contre la carie dentaire.

Toutefois, comme il a été indiqué plus haut en ce qui concerne de façon générale les adhésines de paroi de S. mutans de nature protéique, le couplage de l'antigène 74K SR avec un polysaccharide de S. mutans permet d'obtenir un conjugué ayant des propriétés supérieures.

Le polysaccharide utilisé provient avantageusement de la même souche de S. mutans sérotype f que l'antigène 74K SR. Ce polysaccharide, qui est préparé par exemple selon la méthode de Hamada et coll. indiquée plus haut, est appelé dans ce qui suit "poly f".

Selon un mode de réalisation préféré, le conjugué selon l'invention est constitué de l'antigène 74K SR lié de façon covalente, par amination réductrice, au poly f, après oxydation ménagée de celui-ci.

Quels que soient sa composition et son mode d'obtention, le conjugué selon l'invention peut être utilisé comme antigène, par exemple dans des procédés de séparation d'anticorps ou des dosages.

Il peut en outre être avantageusement utilisé dans des préparations à activité immunologique, notamment pour la prévention des caries dentaires, telles que par exemple des pâtes dentifrice, des chewing-gums etc..

Enfin et surtout, il peut être utilisé comme principe actif dans un vaccin contre la carie dentaire.

Bien que différentes voies d'application du vaccin telles qu'un traitement local ou l'injection puissent être envisagées, il apparaît tout à fait souhaitable que le vaccin se présente sous forme d'une préparation pharmaceutique destinée à l'administration par la voie orale. Il est en particulier souhaitable que cette forme pharmaceutique soit telle qu'elle permette au conjugué d'exercer son action au niveau des plaques de Peyer et des ganglions mésentériques.

Pour ce faire, on peut avantageusement associer le conjugué selon l'invention, ou un mélange de conjugués de ce type, éventuellement en présence d'autres antigènes, avec un adjuvant tel que notamment le muramyldipeptide (MDP) ou des liposomes, selon des techniques connues.

Les formes pharmaceutiques utilisées peuvent comporter différents excipients usuels, notamment à effet retard.

A titre indicatif, une dose de vaccin administrable à l'Homme peut contenir de 1 à 100 mg du conjugué selon l'invention.

Des essais sur l'animal ont montré qu'aux doses efficaces, le conjugué selon l'invention ne présente pas de toxicité.

Il semble qu'une protection plus efficace est obtenue si la vaccination est suivie de plusieurs rappels, par exemple trois, à des intervalles de quelques mois.

L'invention sera mieux comprise à l'aide des exemples détaillés et de la description d'essais pharmacologiques qui suivent.

Exemple 1

Préparation de l'antigène 74K SR

Streptococcus mutans OMZ 175 (sérotype f), dont la préparation est décrite par B. Guggenheim, dans Caries Res. 2, 147-163 (1968) est cultivé en milieu liquide (bouillon coeur-cervelle commercialisé sous la dénomination "Brain-Heart Infusion Broth" de la Société Difco Laboratories, Détroit E.U.A.). Les cellules sont récoltées au bout de 12 heures de culture à 37°C, lavées et extraites avec du tampon phosphate 0,5M, pH 6,0 selon la méthode décrite par M. Schöller et coll. dans Infect. Immun. 31, 52-60 (1981). L'extrait bactérien dans le tampon phosphate est ensuite dialysé selon des méthodes usuelles et utilisé pour la séparation de l'antigène 74K SR.

Cette purification est réalisée grâce à une chromatographie d'immunoaffinité sur colonne de gel d'agarose commercialisé sous la dénomination Sepharose CL 4B (Pharmacia, Uppsala, Suède) activé au bromure de cyanogène et couplé avec un anticorps monoclonal dirigé contre les protéines interagissant avec les glycoprotéines salivaires (soit 8 à 10 mg d'anticorps/ml de gel) dont la préparation est indiquée plus loin. La colonne est équilibrée avec du Tris/HCl 0,01M (pH 7,2)/NaCl 0,15M - (tampon TS).

L'extrait d'antigènes extraits de la paroi cellulaire (WEA) tel que purifié plus haut est chromatographié sur cette colonne. Environ 30 mg de l'extrait dans du tampon Tris contenant 1% de détergent non ionique commercialisé sous la dénomination Triton X-100 et 0,05 % de dodécylsulfate de sodium (SDS) sont appliqués sur la colonne. On rince avec du tampon TS. La matière liée spécifiquement est éluée soit avec du NaCl 5M, soit avec de la glycine/HCl 0,2M, pH 2,8 et est immédiatement neutralisée avec du Tris 1M. Après élimination de l'agent d'élution par dialyse contre du tampon TS à 4°C, on vérifie la pureté de la protéine par électrophorèse en gel de polyacrylamide.

La seule protéine récupérée après élution dans ces conditions particulières a une masse molaire voisine de 74 kDa et représente environ 27 à 31% de l'activité totale de liaison avec les glycoprotéines salivaires des protéines chromatogra-phiées; c'est la protéine 74K SR.

Obtention des anticorps monoclonaux spécifiques des antigènes associés à la paroi cellulaire.

L'extrait d'antigènes associés à la paroi cellulaire est obtenu comme décrit plus haut avec inactivation des protéases selon la méthode de R. R. B. Russell et coll. décrite dans J. Gen. Microbiol. 129, 865-875 (1983). La fraction brute de protéines est chromatographiée sur une colonne d'acrylamide commercialisée sous la dénomination Bio-Gel P6 de la société Bio-Rad Laboratories (Richmond, Ca-lifornie, EUA), et ajustée à une concentration finale en protéines de 2 mg/ml.

Production d'hybridomes sécrétant des anticorps

Des rats de souche Wistar sont immunisés par voie intrapéritonéale avec 50 µg de fraction protéique précipitée à l'alun, deux fois, à 14 jours d'intervalle. Sept mois plus tard, les rats reçoivent un rappel de 100 µg de WEA par voie intraveineuse, dans de la solution saline tamponnée au phosphate (PBS). Les cellules de rate sont recueillies 4 jours plus tard et fusionnées avec des cellules myélomateuses de rats de souche Lou ne sécrétant pas de IR 983F [voir l'article de H. Bazin dans Prot. Biol. Fluids 29, 615-658 (1982)] dans un rapport cellulaire de 5 à 1. La solution de fusion est du polyéthylèneglycol 4000 de la Société Merck - (Darmstadt, RFA) et on fait croître la culture en présence de milieu nutritif de type Eagle modifié dans des puits de plaques de culture. Environ deux semaines après la fusion, on recherche les anticorps contre les antigènes WEA dans les puits ayant subi une croissance de cellules hybrides, par dosage radioimmunologique indirect en phase solide. Les clones positifs sont reclonés dans les conditions usuelles, au moins deux fois, pour assurer la "monoclonicité" et, par transfert sur papier de nitrocellulose (technique dite de "Western Blot"), on sélectionne les hybridomes produisant des anticorps dirigés contre les protéines interagissant avec les glycoprotéines salivaires.

Production de liquide d'ascites et purification des anticorps

Des cellules d'hybridome d'origine monoclonale sont injectées par voie intrapéritonéale à des rats hybrides (Wistar R/Lou C) F1, le liquide d'ascites est recueilli et l'immunoglobuline purifiée.

Chaque anticorps monoclonal de type IgM est précipité à partir du liquide d'ascites clarifié par dialyse contre du tampon Tris-HCl 0, 1M, pH 8 (48 heures, 4°C). L'immunodiffusion radiale des surna-geants révèle une élimination de 100% de l'anticorps à partir de chaque ascite. La protéine est dissoute dans du PBS, pH 7,2, appliquée à une colonne de Bio-Gel A 5m (90 $^\times$ 2,6 cm) de la Société Bio-Rad Laboratories, et éluée avec du tampon PBS. Le pic d'IgM est recueilli, ajusté à une concentration finale en protéines de 4 mg/ml et conservé à -80°C.

Exemple 2

Préparation du polysaccharide de sérotype f (poly f)

Ce polysaccharide est préparé selon la méthode de Hamada et coll. décrite dans Molec. Immunol. 20, 453 (1983) à partir de cellules de S. Mutans OMZ 175 lyophilisées.

En résumé, les cellules sont mises en suspension dans du PBS 7,4 et autoclavées pendant 30 minutes à 120°C. Le surnageant est dialysé contre de l'eau distillée et chromatographié sur une colonne commercialisée sous la dénomination DEAE Trisacryl M de la Société IBF (France), équilibrée avec du tampon Tris/HCl 0,01M (pH 7,4). La matière non-adsorbée est de nouveau chromatographiée sur une colonne de Bio-Gel P100 de la Société Bio-Rad et les fractions éluées dans le volume mort de la colonne, après dialyse contre de l'eau et lyophilisation, sont constituées du polysaccharide de S. Mutans de sérotype f ou poly f.

Exemple 3

Couplage du poly f et de la protéine 74K SR : Obtention du conjugué préféré selon l'invention

Le conjugué est préparé par la méthode de Sanderson et coll. décrite dans Immunology, 20, 1061(1971). 20 mg de poly f sont oxydés par 4 ml de solution 0, 1M de periodate de sodium (pH 4,5) pendant 1 heure, à la température ambiante et dans l'obscurité. Le poly f oxydé est purifié par élution à l'eau sur une colonne de Bio-Gel P100 - (1,6 $^\times$ 20 cm) et lyophilisé. Le poly f oxydé est ensuite couplé à 6 mg d'antigène 74K SR purifié par animation réductrice avec du borohydrure de sodium, selon la méthode de I. Parikh et coll. décrite dans Methods in Enzymology, vol. XXXIV, - (Academic Press) p. 77-102 (1974). Après élimination de l'excès de réactif par chromatographie sur une colonne de Bio-Gel P6 (Bio-Rad), le conjugué brut est appliqué sur une colonne de DEAE Trisacryl M (1,6 × 10 cm) équilibrée avec du tampon Tris/HCl 0,01M (pH 7,4). Après élimination du poly f non couplé, le conjugué poly f-74K SR est élué avec du tampon Tris/HCl 0,01M contenant du NaCl 1M. Cette fraction, après dialyse contre du PBS, est encore débarrassée des protéines n'ayant pas réagi par chromatographie sur un immuno-adsorbant anti poly f. Le conjugué purifié est élué à partir de l'immuno-adsorbant au moyen de tampon acétate 0,1M (pH 4,0) et est immédiatement neutralisé par du tampon phosphate 1M (pH 7,4). Après élimination de l'agent d'élution par chromatographie sur Bio-Gel P6, le conjugué purifié est concentré par lyophilisation.

Sur la base des déterminations des hexoses - [D. L. Morris, Science 107, 254 (1948)] et de la concentration en protéines [O.H. Lowry et coll, J. Biol. Chem.,193, 265 (1951)], on constate que le rapport moléculaire du poly f à la protéine 74K SR dans le conjugué est de 1:0,8.

Etude pharmacologique du conjugué poly f-protéine 74K SR

L'efficacité du conjugué poly f-protéine 74K SR comme antigène a été comparée, au cours d'une expérience sur le rat,d'une part à l'immunogénicité du poly f seul et d'autre part à l'immunogénicité de la protéine 74K SR seule.

Les résultats obtenus montrent que l'immunisation des animaux par le conjugué conduit à une forte réponse immunitaire salivaire de type IgA, dirigée à la fois contre le poly f et la protéine, par opposition à l'absence de réponse immunitaire en IgA salivaires obtenue avec le polysaccharide seul. L'immunogénicité de la protéine couplée est en outre supérieure à celle de la protéine seule.

En conclusion, le couplage d'une adhésine de paroi d'un S. Mutans à un polysaccharide de S. Mutans augmente son antigénicité et conduit à une réponse immunitaire locale forte.

De plus, ce couplage permet d'induire une "mémoire immunologique" contre deux types de récepteurs de surface très importants dans la formation de la plaque dentaire, à savoir des adhésines de nature protéique d'une part, et des adhésines de type polysaccharidique d'autre part.

Enfin, on a pu établir, par la mise en évidence d'une immogénicité croisée, que la protéine 74K SR est commune aux différents sérotypes de S. mutans ou au moins présente un épitope commun avec des protéines de surface des autres sérotypes. En conséquence, un vaccin utilisant un conjugué comprenant cette protéine est efficace contre des souches de S. Mutans de différents sérotypes.

Exemple de préparation d'un vaccin selon l'invention Liposomes contenant le conjugué selon l'invention en tant qu'antigène

La préparation de ces liposomes peut être effectuée selon la méthode de G. Gregoriadis décrite dans Biochem. J. 129, 123 (1971). En particulier, conformément aux indications de D. Wachsmann et coll. dans Immunology 54 (1), 189-194 (1985), le conjugué étant incorporé dans de petits liposomes unilamellaires composés de phosphatidylcholine, de cholestérol et d'acide phosphatidique (rapport molaire 7:2:1).

## Revendications

1. Conjugué constitué d'une adhésine de paroi de Streptococcus mutans, de nature protéique, capable de lier les composants de la salive, ou d'une fraction active d'une telle protéine, couplée de façon covalente à un polysaccharide de Streptococcus mutans, ou à une fraction d'un tel polysaccharide.

2. Conjugué selon la revendication 1, caractérisé en ce que la liaison covalente résulte de l'oxydation ménagée du polysaccharide suivre de l'amination réductrice par la protéine.

3. Adhésine de paroi de S. mutans, de nature protéique utilisable dans la préparation du conjugué selon la revendication 1 ou 2, caractérisée en ce que :

-elle est constituée d'une chaîne simple polypeptidique ayant un poids moléculaire de 74 000 environ;

-elle est commune aux différents sérotypes de Streptococcus mutans où elle présente une antigénicité croisée ; et

-elle est capable d'interagir avec les glycoprotéines salivaires.

4. Conjugué selon le revendication 1 ou 2, catactérisé en ce qu'il est essentiellement constitué de la protéine selon la revendication 3 et d'un polysaccharide provenant de préférence du même sérotype de S. mutans, notamment du sérotype f, et en particulier de la même souche de sérotype f.

5. Procédé pour la préparation du conjugé selon la revendication 2 ou 4, caractérisé en ce qu'il consiste essentiellement à effectuer la liaison covalente par oxydation ménagée du polysaccharide, notamment par le periodate de sodium, suivie d'un couplage avec la protéine par animation réductrice en présence de borohydrure de sodium.

6. Procédé pour la préparation de l'adhésine de paroi selon la revendication 3, caractérisé en ce qu'il consiste essentiellement à extraire des antigènes bruts associés à la paroi cellulaire de bactéries d'une souche de S. mutans de sérotype f au moyen de tampon phosphate 0,5M, pH 6,0 et à purifier la protéine recherchée par chromatographie d'immunoaffinité au moyen d'un anticorps monoclonal dirigé contre les protéines interagissant avec les glycoprotéines salivaires, en présence d'un détergent non ionique et de dodécylsulfate de sodium, avec élution par du tampon NaCl ou glycine/HCl.

7. Utilisation du conjugué selon l'une des revendications 1, 2 ou 4 comme antigène.

8. Composition à activité immunogène comprenant, en tant que principe actif, au moins un conjugué selon l'une des revendications 1, 2 ou 4.

9. Vaccin contre la carie dentaire comprenant comme principe actif au moins un conjugué selon l'une des revendications 1, 2 ou 4.

10. Vaccin selon la revendication 9, caractérisé en ce qu'il se présente sous une forme galénique administrable par voie orale, de préférence en présence d'un adjuvant, tel que notamment le muramyldipeptide ou de préférence encore des liposomes.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | EP-A-0 090 617  (KITASATO KENKYUSHO) ----- | | A 61 K  39/09 |

DOMAINES TECHNIQUES
RECHERCHES (Int. Cl.4)

A 61 K

Le présent rapport de recherche a été établi pour toutes les revendications

| Lieu de la recherche | Date d'achèvement de la recherche | Examinateur |
|---|---|---|
| LA HAYE | 21-07-1986 | REMPP G.L.E. |